# EUROPEAN PATENT APPLICATION

(11) **EP 3 617 741 A1**
(43) Date of publication of application: **04.03.2020**
(21) Application number: 19194472.7
(22) Date of filing: 29.08.2019
(51) Int. Cl.: G01S 7/41, G01S 13/56

(54) **MICROWAVE DETECTION DEVICE AND ITS DETECTION METHOD AND APPLICATION**

(30) Priority: 31.08.2018 CN 201811010625; 29.04.2019 CN 201910318272
(71) Applicant: Shenzhen Merrytek Technology Co., Ltd, 518127 Shenzhen (CN)
(72) Inventor: ZOU, Gaodi, Shenzhen, 518127 (CN); ZOU, Xin, Shenzhen, 518127 (CN); ZOU, Mingzhi, Shenzhen, 518127 (CN)
(74) Representative: Casalonga

(57) **Abstract**

A microwave detection device, which is arranged for detecting movement characteristics of at least one object within a detection area, includes a microwave detecting module generating a difference signal based on a Doppler effect principle and a signal converting module operatively connected to the microwave detecting module. The signal converting module is arranged to convert the differential signal into a wave signal in order to analyze the wave signal for acquiring the movement characteristics of the object within the detection area.

## Description

### BACKGROUND OF THE PRESENT INVENTION

### FIELD OF INVENTION

The present invention relates to a field of microwave detection, and more particularly to a microwave detection device and its detection method and application.

### DESCRIPTION OF RELATED ARTS

It is known that a conventional microwave detector has advantages of high sensitivity, strong anti-interference ability, high reliability, and no effect of ambient temperature. Therefore, the conventional microwave detector is widely used in different industrial production, transportation, and different domestic device such as vehicle speed detector, and detectors for automatic faucets, automatic doors, and smart lighting devices. Accordingly, the conventional microwave detector has a detecting area that when an object moves within the detecting area, such as a person or a vehicle enters into the detecting area, the microwave detector will generate an activation signal or a control signal to the corresponding application for a subsequent operation.

Particularly, the operation of the existing microwave detector is based on microwave Doppler effect principle, wherein the microwave detector emits a microwave detection wave in pulse form or continuous form in the detecting area and, at the same time, receives a reflection wave within the detecting area. When the detection wave encounters a stationary object within the detecting area, there will be no change of the frequency of the reflection wave. When the detection wave encounters a moving object within the detecting area, the frequency of the reflection wave will be changed. According to the frequency difference between the detection wave and the reflection wave, the existing microwave detector further comprises a mixing detector obtaining a Doppler signal corresponding to the amplitude and speed of the moving object. Accordingly, a series of Doppler signals will be generated for each movement of the moving object, wherein the duration of output thereof equals to the duration of the relative motion of the moving object. The speed and amplitude of the moving object are different and the frequency and amplitude of the Doppler signal are also different. Therefore, the microwave detector can effectively detect whether there is a moving object within the detecting area and the speed and amplitude of the moving object.

Accordingly, the existing microwave detector generally processes amplification and analysis of the Doppler signal to identify the movement of the human body. Particularly, after acquiring the Doppler signal, the microwave detector further comprises a signal amplifier for amplifying the Doppler signal in a predetermined magnification, and for filtering out the high frequency signal in the Doppler signal and any interfering signal including the power supply network frequency signal and its multiple frequency signal, so as to minimize the signal interference and to enhance the accuracy of the motion detection of the human body. However, the relevant frequency signal is filtered out and the Doppler signal cannot be over-magnified through such method. As a result, the existing microwave detector cannot detect or monitor any fine action of the human body, such as breathing, heartbeat or fine body movement. In other words, the existing microwave detector only provides a single function that cannot fulfill the application of the intelligence or smart device.

### SUMMARY OF THE PRESENT INVENTION

The invention is advantageous in that it provides a microwave detection device and its detection method and application, wherein the microwave detection device acquires a movement characteristic of an object in a detecting area via the processing of a wave signal, such as continuous slow motion, slow movement with a certain rhythm, fine movement, large movement or high speed movement, and etc., in order to enhance the detection accuracy and practicability of the microwave detecting device.

Another advantage of the invention is to provide a microwave detection device and its detection method and application, wherein the microwave detection device is able to accurately acquire movement characteristics such as body movement, fine movement, respiratory movement, and heartbeat movement of the human body in the detection area.

Another advantage of the invention is to provide a microwave detection device and its detection method and application, wherein different movement characteristics can be determined according to the wave frequency and/or the wave amplitude of the wave signal, so as to improve the detection accuracy of the microwave detection device.

Another advantage of the invention is to provide a microwave detection device and its detection method and application, wherein the microwave detection device is arranged to convert a change of the differential signal into the wave signal, to determine the wave signal corresponding to the movement characteristic of the object in the detection area, and to subsequently process the wave signal to obtain the movement characteristic of the object in the detection area.

Another advantage of the invention is to provide a microwave detection device and its detection method and application, wherein the microwave detection device is arranged to convert a change in amplitude of the differential signal as a reference of the wave signal for subsequent processing the wave signal to obtain the movement characteristic of the object in the detection area.

Another advantage of the invention is to provide a microwave detection device and its detection method and application, wherein the microwave detection device converts a phase change of the differential signal as a reference of the wave signal for subsequent processing the wave signal to obtain the movement characteristic of the object in the detection area.

Another advantage of the invention is to provide a microwave detection device and its detection method and application, wherein the microwave detection device converts a frequency change of the differential signal as a reference of the wave signal for subsequent processing the wave signal to obtain the movement characteristic of the object in the detection area.

Another advantage of the invention is to provide a microwave detection device and its detection method and application, wherein the microwave detection device converts a change of a pulse width of the differential signal as a reference of the wave signal for subsequent processing the wave signal to obtain the movement characteristic of the object in the detection area.

Another advantage of the invention is to provide a microwave detection device and its detection method and application, wherein the microwave detection device converts a frequency change of the differential signal as a reference of the wave signal for subsequent processing the wave signal to obtain the movement characteristic of the object in the detection area.

Another advantage of the invention is to provide a microwave detection device and its detection method and application, wherein the microwave detection device converts the differential signal into the wave signal by means of envelope filtering based on pulse amplitude.

Another advantage of the invention is to provide a microwave detection device and its detection method and application, wherein the microwave detection device converts the differential signal into the wave signal by using a pulse width-based integration method.

Another advantage of the invention is to provide a microwave detection device and its detection method and application, wherein the microwave detection device is able to determine the movement characteristics of the object in the detection area by selecting the wave signal in a specific frequency range.

Another advantage of the invention is to provide a microwave detection device and its detection method and application, wherein the wave signal is amplified by a signal amplifying module to more accurately obtain the specific movement characteristics of the object in the detection area.

Another advantage of the invention is to provide a microwave detection device and its detection method and application, wherein the signal amplifying module is arranged to amplify the differential signal with a weak signal for accurate identification and calculation to obtain the wave signal and to enhance the detection accuracy of the microwave detection device.

Another advantage of the invention is to provide a microwave detection device and its detection method and application, wherein the wave signal is selected to have a frequency range of 50 Hz or less for minimizing the interference of the wave signal by different environmental factors, for example, but not limited to, electromagnetic radiation generated by the power supply network and other electrical circuits.

Another advantage of the invention is to provide a microwave detection device and its detection method and application, wherein the wave signal is selected to have a frequency range 25 Hz or less to further minimize the wave signal from being interfered by other environmental factors, such as, but not limited to, the electromagnetic radiation generated by the power supply network and other electrical circuits. Therefore, the wave signal can be amplified by several tens of times, hundreds of times, thousands of times or even tens of thousands of times without being interfered, so as to obtain any weak or fine motion signal of, such as, body movement, fine movement, respiratory movement, and heartbeat movement of the human body in the detection area.

Another advantage of the invention is to provide a microwave detection device and its detection method and application, which is suitable for the signal amplifying module within 25 Hz, wherein the signal amplifying module is operated in an ultra-low frequency range. Within this ultra-low frequency range, there are very few signal sources of electromagnetic radiation and other interfering signals in this environment, such that an electromagnetic radiation free environment is provided to improve the accuracy and stability of the microwave detection device.

Another advantage of the invention is to provide a microwave detection device and its detection method and application, wherein the signal of the heartbeat movement and the respiratory movement of the human body in the detection area can be obtained by selecting the wave signal having a frequency of 3 Hz or less.

Another advantage of the invention is to provide a microwave detection device and its detection method and application, wherein the signal of the heartbeat movement of the human body in the detection area can be obtained by selecting the wave signal having a frequency of 1 Hz - 3 Hz.

Another advantage of the invention is to provide a microwave detection device and its detection method and application, wherein the signal of the respiratory movement of the human body in the detection area can be obtained by selecting the wave signal having a frequency of 1 Hz or less.

Another advantage of the invention is to provide a microwave detection device and its detection method and application, wherein the signal amplifying module is able to amplify any signal of a weak or fine movement such as the respiratory movement, and heartbeat movement of the human body in tens of times, hundreds of times, thousands of times or even tens of thousands of time of magnifications in order to more accurately obtain the movement characteristics of the human body.

Another advantage of the invention is to provide a microwave detection device and its detection method and application, wherein when the microwave detection device is used for detecting human body activity, the detected differential signal is converted into the wave signal, wherein the frequency of the wave signal corresponding to the movement characteristic of the human body in the wave signal is in the low frequency range to minimize any interferences in the environment, such as vibration generated by air conditioners and exhaust fans, malfunction caused by small animals, interference from wind and rain, and etc., so as to improve the accuracy and stability of the microwave detection device.

Another advantage of the invention is to provide a microwave detection device and its detection method and application, wherein the microwave detection device comprises a microwave emitter and a reflecting wave receiving unit. The microwave emitter is arranged to emit a detection wave in the detection area, wherein the reflecting wave receiving unit is arranged to receive the reflected wave corresponding to the detection wave. When the object is moving in the detection area, the frequency and phase of the detection wave and the reflected wave are different, such that the difference in frequency and/or phase of the detection wave and the reflected wave is subsequently process and analyzed to obtain the differential signal.

Another advantage of the invention is to provide a microwave detection device and its detection method and application, wherein the microwave detection device comprises a processing unit arranged to obtain the differential signal in response to the frequency and/or phase difference of the detection wave and the reflected wave.

Another advantage of the invention is to provide a microwave detection device and its detection method and application, wherein the microwave detection device comprises a signal converting module arranged to convert the differential signal into the wave signal and to subsequently process the wave signal for acquiring specific movement characteristics of the object in the detection area.

Another advantage of the invention is to provide a microwave detection device and its detection method and application, wherein the signal converting module is arranged to select the wave signal to subsequently acquire the specific movement characteristics of the object in the detection area.

Another advantage of the invention is to provide a microwave detection device and its detection method and application, wherein the signal amplifying module of the microwave detection device is arranged to amplify the wave signal for accurately measuring and identifying of the wave signal and for more accurately acquiring the movement characteristics of the object in the detection area to improve the accuracy of the microwave detection device.

Another advantage of the invention is to provide a microwave detection device and its detection method and application, wherein the microwave detection device is able to incorporate with a smart device to enable the smart device being automatically controlled and adjusted for providing any corresponding intelligent service based on the object in the detection area.

Additional advantages and features of the invention will become apparent from the description which follows, and may be realized by means of the instrumentalities and combinations particular point out in the appended claims.

According to the present invention, the foregoing and other objects and advantages are attained by a microwave detection device having a detection area for detecting movement characteristics of at least one object therewithin, comprising:
a microwave detecting module generating a differential signal based on Doppler effect principle; and
a signal converting module operatively connected to the microwave detecting module, wherein the signal converting module converts the difference signal into a wave signal being analyzed for determining movement characteristics of the object in the detection area.

In one embodiment, the microwave detecting module is arranged to emit the detection wave in the detecting area, wherein the reflected wave is formed when the detection wave is reflected by the object in the detecting area. The differential signal is outputted by the microwave detecting module in response to the frequency difference between the detection wave and the reflected wave.

In one embodiment, the microwave detecting module is arranged to emit the detection wave in the detecting area, wherein the reflected wave is formed when the detection wave is reflected by the object in the detecting area. The differential signal is outputted by the microwave detecting module in response to the phase difference between the detection wave and the reflected wave.

In one embodiment, the signal converting module is arranged to convert the differential signal into the wave signal in response to a properties change thereof for determining the movement characteristics of the object in the detection area.

In one embodiment, the signal converting module is arranged to convert the differential signal into the wave signal in response to an amplitude change thereof for determining the movement characteristics of the object in the detection area.

In one embodiment, the signal converting module is arranged to convert the differential signal into the wave signal in response to a phase change thereof for determining the movement characteristics of the object in the detection area.

In one embodiment, the signal converting module is arranged to convert the differential signal into the wave signal in response to a change in a pulse width thereof for determining the movement characteristics of the object in the detection area.

In one embodiment, the signal converting module is arranged to convert the differential signal into the wave signal in response to a frequency change thereof for determining the movement characteristics of the object in the detection area.

In one embodiment, the signal converting module is arranged to output the wave signal in a specific frequency range.

In one embodiment, the specific frequency range is set as 50 Hz or less.

In one embodiment, the specific frequency range is set as 25 Hz or less.

In one embodiment, the specific frequency range is set as 1 Hz or less.

In one embodiment, the specific frequency range is set as 3 Hz or less.

In one embodiment, the specific frequency range is set between 1-3 Hz.

In one embodiment, the signal converting module is embodied as an analog filter.

In one embodiment, the signal converting module is embodied as a digital filter.

In one embodiment, the signal converting module is embodied as an integrated filter integrally constructed by an analog filter and a digital filter.

In one embodiment, the microwave detection device comprises at least one signal amplifying module operatively connected to the microwave detecting module and the signal converting module, wherein the differential signal is amplified by the signal amplifying module and is then converted into the wave signal by the signal converting module.

In one embodiment, the microwave detection device comprises at least one signal amplifying module operatively connected to the signal converting module, wherein the differential signal is amplified by the signal amplifying module and is then converted into the wave signal by the signal converting module.

In one embodiment, the microwave detection device comprises at least one signal amplifying module for amplifying the differential signal, wherein the wave signal is amplified by at least one of the signal amplifying modules.

In one embodiment, the microwave detection device further comprises a central processing module operatively connected to the signal converting module, wherein the central processing module is arranged to process and analyze the wave signal in order to determine the movement characteristics of the object in the detection area through the wave signal.

In one embodiment, the central processing module is arranged to determine different movement characteristics in response to a wave frequency and/or a wave amplitude of the wave signal.

In one embodiment, the central processing module is arranged to determine different movement characteristics in response to different phase differences of the wave signal.

In one embodiment, the central processing module is arranged to determine different movement characteristics in response to time duration of the wave signal.

In one embodiment, the central processing module is arranged to determine different movement characteristics in response to an amplitude change of the wave signal in one cycle.

In one embodiment, the central processing module comprises a signal sampling unit, a data processing unit and an output unit operatively connected with each other. Preferably, the signal sampling unit, the data processing unit and the output unit are integrated to form a single integral unit.

In one embodiment, the signal amplifying module, and the signal sampling unit, the data processing unit and the output unit of the central processing module are integrated to form a single integral unit.

In one embodiment, the signal converting module, and the signal sampling unit, the data processing unit and the output unit of the central processing module are integrated to form a single integral unit.

In one embodiment, the signal converting module, the signal amplifying module, and the signal sampling unit, the data processing unit and the output unit of the central processing module are integrated to form a single integral unit.

In one embodiment, the microwave detecting module and the signal amplifying module are integrated to form a single integral unit.

In one embodiment, the microwave detecting module, the signal amplifying module and the signal converting module are integrated to form a single integral unit.

In one embodiment, the microwave detecting module, the signal converting module, the signal amplifying module, and the signal sampling unit, the data processing unit, and the output unit of the central processing module are integrated to form a single integral unit.

In one embodiment, the signal converting module and the signal amplifying module are integrated to form a single integral unit.

In accordance with another aspect of the invention, the present invention comprises a smart device, comprising:
at least one microwave detection device having a detection area for detecting movement characteristics of the object therewithin, wherein the microwave detection device comprises:
   a microwave detecting module generating a differential signal; and
   a signal converting module operatively connected to the microwave detecting module, wherein the signal converting module is arranged to convert the difference signal into a wave signal being analyzed for determining the movement characteristics of the object in the detection area;
an execution circuit module operatively connected to the microwave detection device; and
a device body, wherein the execution circuit module is operatively connected to the device body, wherein the execution circuit module is arranged to control the device body in response to the movement characteristic of the object in the detection area obtained by the microwave detection device.

In accordance with another aspect of the invention, the present invention comprises a detection method via the microwave detection device, wherein the detection method comprises the following steps.
(a) Convert a differential signal to a wave signal, wherein the wave signal corresponds to a movement characteristic of an object within a detection area.
(b) Process the wave signal to acquire the movement characteristic of the object in the detection area.

In one embodiment, before the step (a), the detection method further comprises the steps of:
transmitting or emitting the detection wave to the detection area and receiving the reflected wave corresponding to the detection wave; and
outputting the differential signal according to a frequency and/or phase difference of the detection wave and the reflected wave.

In one embodiment, in the step (a), the differential signal is converted to the wave signal in response to a properties change of the differential signal.

In one embodiment, the differential signal is converted to the wave signal in response to the amplitude change of the differential signal.

In one embodiment, the differential signal is converted to the wave signal in response to the phase change of the differential signal.

In one embodiment, the differential signal is converted to the wave signal in response to the change in pulse width of the differential signal.

In one embodiment, the differential signal is converted to the wave signal in response to the frequency change of the differential signal.

In one embodiment, in step (a), the differential signal is converted to the wave signal by using an envelope filtering method based on pulse amplitude.

In one embodiment, in step (a), the differential signal is converted to the wave signal by means of a pulse width based integration method.

In one embodiment, in the step (a), the movement characteristic of the object in the detection area is acquired by selecting the wave signal in a specific frequency range.

In one embodiment, according to the detection method, the specific frequency range of the wave signal is selected and limited to be 50 Hz or less in order to filter the wave signal at other frequency segments.

In one embodiment, according to the detection method, the specific frequency range of the wave signal is selected and limited to be 25 Hz or less in order to filter the wave signal at other frequency segments.

In one embodiment, according to the detection method, the specific frequency range of the wave signal is selected and limited to be 3 Hz or less in order to filter the wave signal at other frequency segments.

In one embodiment, according to the detection method, the specific frequency range of the wave signal is selected and limited between 1-3 Hz in order to filter the wave signal at other frequency segments.

In one embodiment, according to the detection method, the specific frequency range of the wave signal is selected and limited to be 1 Hz or less in order to filter the wave signal at other frequency segments.

In one embodiment, the method further comprises a step of amplifying the differential signal before the step (a).

In one embodiment, the method further comprises a step of amplifying the differential signal after the step (a).

In one embodiment, before and after the step (a), the method further comprises a step of amplifying the differential signal and the wave signal respectively.

In one embodiment, according to the detection method, the differential signal is converted into the wave signal by an analog filter and, at the same time, the wave signal at the specific frequency is selected.

In one embodiment, according to the detection method, the peak value and the average value of the differential signal are extracted by the digital filter by using a filtering algorithm, wherein the peak value or the average value is then combined together to form the wave signal.

In one embodiment, according to the detection method, different movement characteristics of the object in the detection area are determined in response to the wave frequency and/or the wave amplitude of the wave signal.

In one embodiment, according to the detection method, different movement characteristics of the object in the detection area are determined in response to the phase differences of the wave signals.

In one embodiment, in the step (b), different movement characteristics of the object in the detection area are determined in response to the time duration of the wave signal.

In one embodiment, in the step (b), different movement characteristics of the object in the detection area are determined in response to the amplitude change of the wave signal in one cycle.

### BRIEF DESCRIPTION OF THE DRAWINGS

Fig. 1 is a block diagram of a microwave detection device according to a first preferred embodiment of the present invention.
Fig. 2 is a circuit diagram of a signal amplifying module of the microwave detection device according to the above first preferred embodiment of the present invention.
Fig. 3A is a circuit diagram of a signal converting module of the microwave detection device according to the above first preferred embodiment of the present invention.
Fig. 3B is a block diagram of the signal converting module of the microwave detection device according to the above first preferred embodiment of the present invention.
Fig. 4A is a schematic diagram, illustrating the signal converting module converting a differential signal into a wave signal when the microwave detection device is used for detecting the breathing movement of the human body, according to the above first preferred embodiment of the present invention.
Fig. 4B is a schematic diagram, illustrating the signal converting module converting a differential signal into a corresponding wave signal, according to the above first preferred embodiment of the present invention.
Fig. 5A illustrates an application of the microwave detection device for detecting a high speed moving vehicle according to the above first preferred embodiment of the present invention.
Fig. 5B illustrates the signal converting module converting the differential signal into the corresponding wave signal when the microwave detection device is used for detecting the high speed moving vehicle according to the above first preferred embodiment of the present invention.
Fig. 6 is a block diagram of a microwave detection device according to a second preferred embodiment of the present invention.
Fig. 7 is a block diagram of a microwave detection device according to a third preferred embodiment of the present invention.
Fig. 8 is a block diagram of a microwave detection device according to a fourth preferred embodiment of the present invention.
Fig. 9 is a block diagram, illustrating an application of the microwave detection device, according to the above preferred embodiments of the present invention.

### DETAILED DESCRIPTION OF THE PREFERRED EMBODIMENT

The following description is disclosed to enable any person skilled in the art to make and use the present invention. Preferred embodiments are provided in the following description only as examples and modifications will be apparent to those skilled in the art. The general principles defined in the following description would be applied to other embodiments, alternatives, modifications, equivalents, and applications without departing from the spirit and scope of the present invention.

It is appreciated that the terms "longitudinal", "transverse", "upper", "lower", "front", "rear", "left", "right", vertical", "horizontal", "top", "bottom", "exterior", and "interior" in the following description refer to the orientation or positioning relationship in the accompanying drawings for easy understanding of the present invention without limiting the actual location or orientation of the present invention. Therefore, the above terms should not be an actual location limitation of the elements of the present invention.

It is appreciated that the terms "one", "a", and "an" in the following description refer to "at least one" or "one or more" in the embodiment. In particular, the term "a" in one embodiment may refer to "one" while in another embodiment may refer to "more than one". Therefore, the above terms should not be an actual numerical limitation of the elements of the present invention.

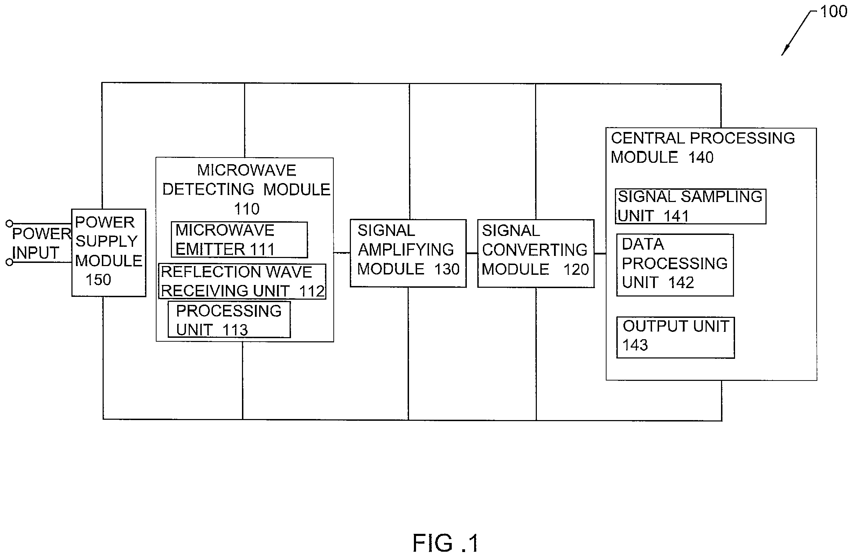

Referring to Figs. 1 to 4A, a microwave detection device 100 according to a first preferred embodiment of the present invention is illustrated. The microwave detection device 100 has a detection area and is adapted for detecting any object movement in the detection area, wherein the microwave detection device 100 is configured to process a wave signal 101 when an object moves within the detection area. The movement of the object can be, but limited to, a continuous slow motion, a slow motion with a predetermined rhythm, a small amplitude movement, a large amplitude movement, a high speed movement, and etc.. In other words, the microwave detection device 100 of the present invention is able to comprehensively detect any motion within the detection area to enhance the accuracy and the practicability of the microwave detection device 100. Accordingly, the microwave detection device 100 is arranged to convert a change of differential signal 102 into a wave signal 101. In response to the characteristics of the movement within the detection area, the signal will be processed to acquire such characteristics of the movement. It is worth mentioning that, through this method, the characteristics of the movement, such as human movement, fine movement, breathing motion, and/or heartbeat motion, within the detection area can be effectively achieved.

As shown in Fig. 1, the microwave detection device 100 comprises a microwave detecting module 110 and a signal converting module 120 communicatively connected to the microwave detecting module 110. The microwave detecting module 110 is arranged to output the differential signal 102, wherein the signal converting module 120 is arranged to receive the differential signal 102 and to convert the differential signal 102 into a wave signal 101 corresponding to the characteristics of the movement within the detection area. Furthermore, the signal converting module 120 is arranged to select the wave signal 101 to obtain specific movement characteristics of the object within the detection area.

As shown in Fig. 1, the microwave detection device 100 further comprises at least a microwave emitter 111, a reflection wave receiving unit 112 and at least a processing unit 113, wherein the processing unit 113 is operatively connected to the microwave emitter 111 and the reflection wave receiving unit 112. Accordingly, the microwave emitter 111 is arranged to emit a detection wave in the detection area, wherein the reflection wave receiving unit 112 is arranged to receive the reflection wave corresponding to the detection wave. When the object moves within the detection area, the frequency between the detection wave and the reflection wave is changed. The processing unit 113 obtains the differential signal 102 corresponding to the frequency difference or change and/or a phase difference between the detection wave and the reflection wave. Preferably, the processing unit 113 is implemented as a mixing detector arranged to detect the phase change between the detection wave and the reflection wave so as to generate the differential signal. Alternatively, there are two processing units 113 that one of the processing units 113 obtains a first intermediate data corresponding to the frequency and/or phase of the detection wave and a reference wave, while another processing unit 113 obtains a second intermediate data corresponding to the frequency and/or phase of the reflection wave and the reference wave. Then, the differential signal 102 will be generated from the first and second intermediate data. It should be understood by the person who skilled in the art that the implementation of the processing unit 113 and the implementation method of obtaining the differential signal are merely examples which should not be limited in the present invention.

It is worth mentioning that the implementation of using the frequency of the microwave for detecting the movement of the object in the detecting area should not be limited in the present invention. Preferably, the frequency of the microwave can be 5.8 GHz, 10.525 GHz, or 24.125 GHz of ISM band. The higher the frequency of the microwave source, the more sensitive the response to the weak or fine motion. The higher the frequency of the differential signal 102, the better the improvement of the resolution of the differential signal 102. Therefore, the accuracy of the microwave detection device 100 will be enhanced.

Furthermore, the signal converting module 120 is arranged to convert the differential signal 102 into the wave signal 101 based on the change of the differential signal 102. The differential signal 102 contains the characteristics of the motion corresponding to the object movement within the detecting area. Preferably, the signal converting module 120 is arranged to convert the amplitude change of the differential signal 102 into the reference of the waver signal 101. Alternatively, the signal converting module 120 is arranged to convert the phase change of the differential signal 102 into the reference of the waver signal 101. Alternatively, the signal converting module 120 is arranged to convert the pulse width change of the differential signal 102 into the reference of the waver signal 101. Alternatively, the signal converting module 120 is arranged to convert the frequency change of the differential signal 102 into the reference of the waver signal 101.

It is worth mentioning that through this method, the microwaves in different frequencies are reflected by the same object in the detection area to form the reflection wave. The processing unit 113 is arranged to receive the differential signal 102 corresponding to the difference between microwave and the reflection wave. After the differential signal 102 passes through the signal converting module 120, the wave signal 101 is consistent after the conversion. Furthermore, the parameters of the wave signal 101 are corresponding to different motion characteristics of the object in the detection area.

According to the preferred embodiment, the signal converting module 120 performs an envelope processing on the differential signal 102 in order to obtain the wave signal 101. For example, the signal converting module 120 is arranged to convert the differential signal 102 into the wave signal 101 by using an envelope filtering method based on the pulse amplitude. Preferably, the signal converting module 120 is arranged to convert the differential signal 102 into the wave signal 101 by using a pulse width based on the integration method.

It is apparent that person who skilled in the art should understand that the implementation of the conversion of the differential signal 102 into the wave signal 101 is merely an example and should not be limited for the signal processing of the microwave detector 100 of the present invention.

Furthermore, the signal converting module 120 is arranged to acquire the motion characteristics of the object within the detecting area by selecting specific frequency range of the wave signal 101. In one example, the microwave detection device 100 is used for detecting the user's daily life or the user in a working environment. The normal respiratory movement of an adult is about 0.2-0.4 times per second, and the normal respiratory movement of a newborn baby is about 0.33-0.75 times per second. Sickness may increase or decrease the respiratory movement within 1 time per second. By setting the relevant parameters of the signal converting module 120, the wave signal 101 is filtered to filter out unrelated frequency segments thereof in order to select the wave signal 101 within a specific frequency range of 1 Hz for detecting the signal of respiratory movement of the user. Under a quiet or calm state, the heartbeat movement of the human body is about 1.0-1.7 times per second, and particularly in a range of 1.0-1.3 times per second. The heartbeat movement of a child under 3 years old is between 1.3 times per second and 2.5 times per second. By setting the relevant parameters of the signal converting module 120, the wave signal 101 is filtered to filter out unrelated frequency segments thereof in order to select the wave signal 101 within a specific frequency range of 1-3 Hz for detecting the signal of heartbeat movement of the user.

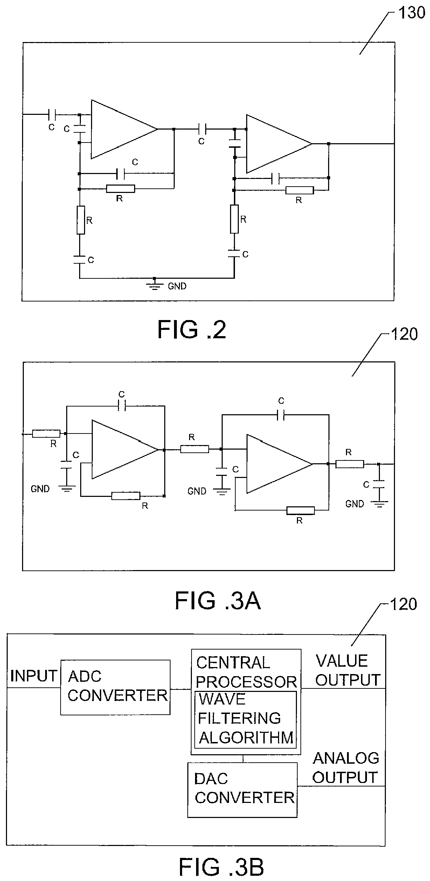

Fig. 3A illustrates a specific embodiment of the microwave detection device 100 of the present invention. The signal converting module 120 is embodied as an analog filter constructed to include at least a capacitor, at least a resistor, at least an inductor, and at least an integrated filtering circuit. For the resistor-capacitor (RC) configuration, the differential signal 102 is integrated and configured to have an average value when the differential signal 102 passes through the analog filter (signal converting module 120) in order to form the wave signal 101 having gradually increasing and decreasing tendencies. At the same time, the analog filter 120 output the wave signal 101 as an analog signal. By setting the analog filter 120 at different parameters, the wave signal 101 is filtered to effectively filter out the unrelated frequency range so as to select the wave signal at the desired frequency range. For example, after the differential signal 102 passes through the signal converting module 120, the unrelated frequency segments, such as ultra-high frequency signals and high frequency signals, are filtered out of the differential signal 102 while the necessary and useful frequency segments, such as ultra-low frequency signals and low frequency signals, are remained in the differential signal 102. In other words, the signal converting module 120 in this example is an analog low pass filter.

It is worth mentioning that the analog filter 120 should not be limited that the analog filter 120 can be constructed with a LC configuration and/or a RC configuration to form a low pass filter, a high pass filter, a band pass filter, a band resistor filter, a dielectric filter, an active filter, a passive filter or any combination of one or more known filters. It should be understood by those skilled in the art that the specific implementation of the analog filter 120 is merely an example and should not be limited in the content and scope of the microwave detection device 100 and the differential signal 102 processing method of the present invention.

Particularly, the signal converting module 120 can be a Butterworth low pass filter to convert the differential signal 102 into the wave signal 101 based on the integrated characteristics of the Butterworth low pass filter and to selectively obtain the wave signal 101 in a particular frequency range via the parameters of the Butterworth low pass filter.

In another embodiment, the signal converting module 120 is embodied as a digital filter, wherein digital filter 120 is arranged to obtain the wave signal 101 by extracting numerical values of the differential signal 102. Preferably, after amplifying the differential signal 102, the peak value or the average value of the amplified differential signal 102 is extracted by using a filtering algorithm, wherein the wave signal 101 is formed corresponding to the change of the peak or the average value. At the same time, the signal converting module 120 is arranged to output the differential signal 102 as the digital signal. Accordingly, the digital filter 120 is operated through a computer via a corresponding program and algorithm in order to perform the filtering process for the digital filter 120. By setting the digital filter 120 at different parameters, the unrelated frequency segments will be filtered out of the wave signal 101 and the necessary and useful frequency segments will be remained in the wave signal 101 correspondingly.

Fig. 3B illustrates another specific embodiment of the microwave detection device 100 of the present invention. The signal converting module 120 is embodied to include an ADC conversion module (embodied as ADC converter), a central processor and a digital low pass filter of a DAC conversion module (embodied as DAC converter), wherein the ADC conversion module, the central processor and the DAC conversion module are communicatively connected with each other. The central processor is arranged to execute the digital filter algorithm. Alternatively, the ADC conversion module and the DAC conversion module are integrated with the central processor. It should be understood that the specific hardware configuration and algorithm of the digital filter should not be limited. For example, but not limited to, the digital filter can be configured to support an MCU, DSP, FPGA, external high precision ADC integrated chip, a digital logic unit chip with an operational amplifier, or any known chips in the art. The corresponding algorithm can be, but are not limited to, Butterworth filter algorithm to convert the differential signal 102 into the wave signal 101 based on the integrated characteristics of the Butterworth low pass filter and to selectively obtain the wave signal 101 in a particular frequency range via the parameters of the Butterworth low pass filter. Alternatively, the algorithms can be Fourier (FFT/DFT) algorithm, Kalman filter algorithm, finite impulse response filter, non-recursive filter (FIR) algorithm, Hilbert-Huang Transform (HHT), linear system transform, wavelet transform, infinite impulse response filter, infinite impulse response filter (IIR) algorithm or any known algorithms in the art.

It is worth mentioning that when the signal converting module 120 is implemented as the Butterworth low-pass filter, the digital filter is implemented with the Butterworth algorithm. For example, a first order Butterworth filter or a multi-order Butterworth filter are configured in a series connection or a parallel connection. Based on the integrated characteristics of the Butterworth low pass filter, the interference of the differential signal 102, such as a small object with a high speed motion under the high wind condition or raining condition, will be converted into the wave signal 101 with small fluctuation so as to process as a background noise by the existing signal processing techniques. In other words, the interference of the wave signal 101, i.e. corresponding to the small object with a high speed motion under the high wind condition or raining condition, will be ignored to enhance the accuracy and stability of the detection of the moving human body and other objects by the microwave detection device 100. Thus, the microwave detection device 100 is suitable for detecting a moving object such as a vehicle in an outdoor environment.

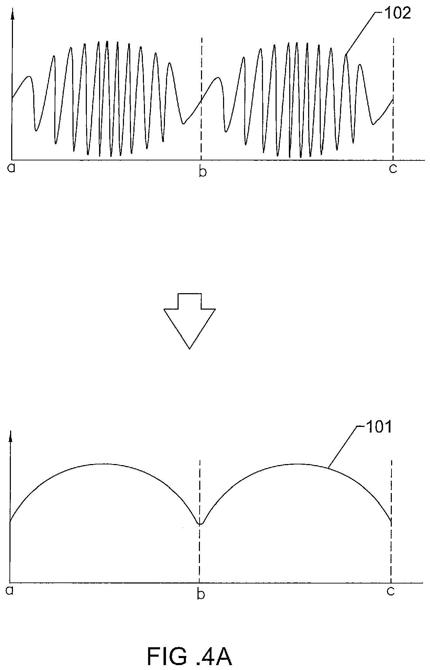

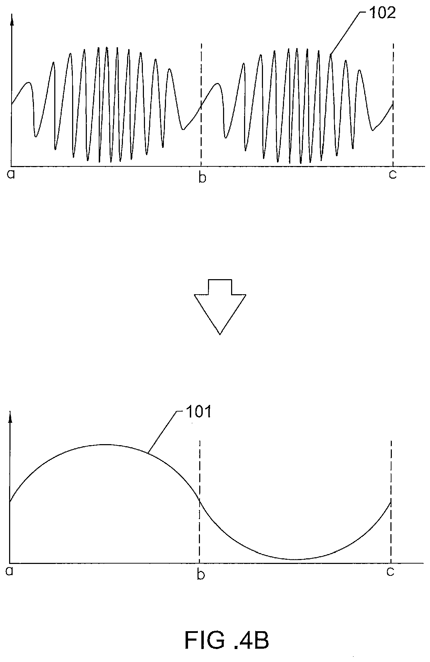

Figs. 4A and 4B illustrate an example of the microwave detection device 100 for detecting the respiratory movement of the user, wherein the respiratory movement of the user is defined as the chest of the user outwardly expands from a first point "a" to the maximum point "b". After amplifying the differential signal 102, the amplitude of the amplified differential signal 102 is initially changed from low to high, and then from high to low. The frequency of the amplified differential signal 102 is initially changed from low to high, and then from high to low. The respiratory movement of the user is further defined as the chest of the user inwardly contracts from the maximum point "b" to the third point "c". The amplitude of the amplified differential signal 102 is initially changed from low to high, and then from high to low. The frequency of the amplified differential signal 102 is initially changed from low to high, and then from high to low. The wave period of the respiratory movement of the user is completed at the point "c". The wave signal 101 can be obtained through the above mentioned software filter, integrated averaging method, Fourier (FFT/DFT) algorithm, Butterworth filter algorithm, Kalman filter algorithm, finite impulse response filter, non-recursive filter (FIR) algorithm, Hilbert-Huang Transform (HHT), linear system transform, wavelet transform, infinite impulse response filter, and/or infinite impulse response filter (IIR) algorithm. It is worth mentioning that the shape, period, amplitude, width of the differential signal 101 and the wave signal 102 are shown in Figs. 4A and 4B for illustrative purpose, and should not particularly represent the actual respiratory movement of the human being. The example should not be limited in the content and range of the microwave detection device 100 and the microwave detection method of the present invention.

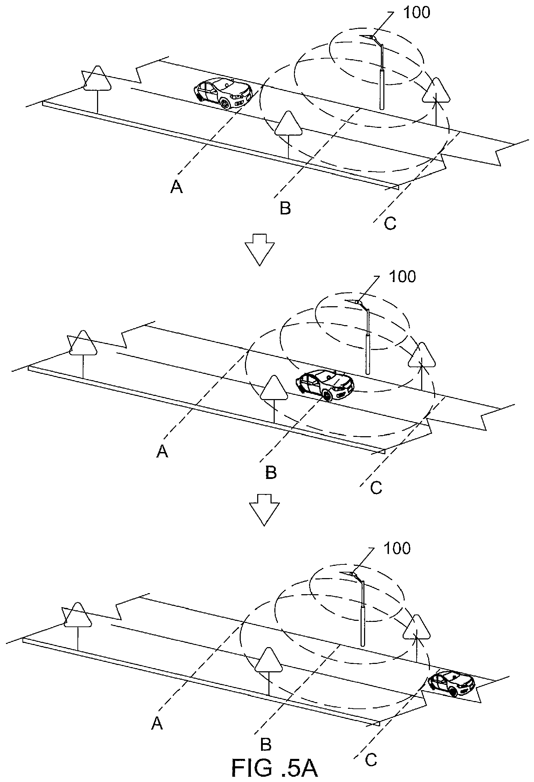

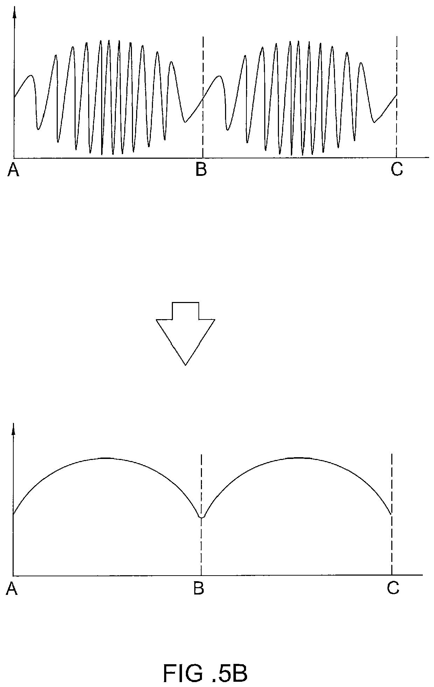

Figs. 5A and 5B illustrate another example of the microwave detection device 100 for detecting the high speed moving vehicle, wherein the microwave detection device 100 can be installed in a street light pole beside a road. When the vehicle enters into the detection area from a point A toward the stationary microwave detection device 100 at a point B, the amplitude of the amplified differential signal 102 is initially changed from low to high, and then from high to low. The frequency of the amplified differential signal 102 is initially changed from low to high, and then from high to low. After passing the point B and moving away from the microwave detection device 100, the amplitude of the amplified differential signal 102 is initially changed from low to high, and then from high to low. The frequency of the amplified differential signal 102 is initially changed from low to high, and then from high to low. When the vehicle leaves the detection area at a point C, the wave period of the differential signal 102 is completed. The wave signal 101 can be obtained through the above mentioned software filter, integrated averaging method, Fourier (FFT/DFT) algorithm, Butterworth filter algorithm, Kalman filter algorithm, finite impulse response filter, non-recursive filter (FIR) algorithm, Hilbert-Huang Transform (HHT), linear system transform, wavelet transform, infinite impulse response filter, and/or infinite impulse response filter (IIR) algorithm. It is worth mentioning that the shape, period, amplitude, width of the differential signal 101 and the wave signal 102 are shown in Figs. 5A and 5B for illustrative purpose, and should not particularly represent the actual moving vehicle. The example should not be limited in the content and range of the microwave detection device 100 and the microwave detection method of the present invention.

It should be understood by those skilled in the art that the implementation of the signal converting module 120 is merely an example and should not be limited in the content and scope of the microwave detection device 100 and the differential signal processing method of the present invention. In another embodiment of the present invention, the signal converting module 120 may be implemented as an integrated digital filter and analog filter. For example, but not limited to, the chip is integrated with digital filtering and analog filtering circuits.

According to a preferred embodiment, the microwave detection device 100 further comprises at least a signal amplifying module 130 communicatively connected to the signal converting module 120, wherein the signal amplifying module 130 is arranged to specifically amplify the wave signal 101 in different frequency ranges at a specific magnification in order to obtain a specific characteristics movement of the object in the detection area. In addition, the signal amplifying module 130 is able to precisely determine the weak wave signals 101, discontinuous wave signals 101, or irregular wave signals 101, so as to minimize any interference during the subsequent analysis of the wave signal 101. As a result, the microwave detection device 100 is able to accurately acquire specific characteristics of the movement of the object in the detection area.

Preferably, the specific frequency range selected by the signal converting module 120 is smaller than the frequency of the power supply network. In other words, the frequency of the wave signal 101 passing through the signal converting module 120 is smaller than the frequency of the power supply network. Therefore, the electromagnetic radiation generated by the power supply line and other electric circuits would not interfere to the wave signal 101, so as to allow the wave signal 101 to be amplified by tens of times, hundreds of times, thousands of times, or even tens of thousands of times without affecting the accuracy of the wave signal 101. Preferably, the specific frequency range selected by the signal converting module 120 is within 50 Hz. In other words, the frequency of the wave signal 101 is set less than 50 Hz. Therefore, the electromagnetic radiation generated by the power supply line and the interference of other electric circuits can be effectively minimized to enhance the detection accuracy of the microwave detection device 100. It should be understood by those skilled in the art that the specific frequency range is merely an example and should not be limited in the content and scope of the microwave detection device 100 of the present invention. In addition, it should be understood that different frequencies of the power supply networks are used in different countries, such that different frequency ranges should be set according to the frequency of the power supply networks in different environments.

Preferably, the specific frequency range selected by the signal converting module 120 is set at 25 Hz or less, and the signal amplifying module 130 is embodied as an amplifier suitable for various amplifiers within 25 Hz. The amplifier 130 is set to operate at an ultra-low frequency range no matter the signal is magnified at tens of times, hundreds of times, thousands of times, or even tens of thousands of times. Since the frequency of the wave signal 101 is lower than one time of the frequency of the power supply network, it will not be interfered by other environmental factors under a normal electromagnetic environment, so as to enhance the accuracy of the microwave detection device 100.

In other words, the signal amplifying module 130 is operated at an ultra-low frequency range. Accordingly, electromagnetic radiation and other interfering signals are rarely found within the ultra-low frequency range. Under the electromagnetic radiation free environment, the signal amplifying module 130 is able to amplify the wave signal 101 at multiple amplifications to obtain an accurate and useful wave signal 101, so as to enhance the accuracy and stability of the microwave detection device 100. For example, the wave signal 101 corresponds to the frequency of the wave signal of the human body is relatively low, and the fine motion such as the breathing motion and the heartbeat motion of the human body is amplified to a useful magnitude, so as to accurately acquire the motion characteristics of the human body. It is worth mentioning that when the microwave detection device 100 detects human activity or movement, the detected differential signal 102 is converted into the wave signal 101. Since the frequency of the wave signal 101 corresponding to the motion characteristic of the human body is in a low frequency range, it can avoid other interferences in the surrounding environment, such as the vibration caused by the air conditioner and the exhaust fan, the malfunction caused by small animals, the interference of wind and rain, and etc.. As a result, the accuracy of the microwave detection device 100 can be enhanced.

According to a preferred embodiment, the signal amplifying module 130 is operatively connected to the processing unit 113 of the microwave detecting module 110 and the signal converting module 120. The signal amplifying module 130 is arranged to amplify the differential signal 102, such that the relatively weak differential signal 102 can also be amplified for being accurately identified and calculated by the signal converting module 120 during subsequent processing, so as to enhance the accuracy of the microwave detection device 100. For example, the frequency range of the differential signal 102, corresponding to the movement characteristics of the human body movement, limb movement, and the vehicle movement, is low. Accordingly, the frequency range of the differential signal 102 is only between 0.0001 Hz and 200 Hz, such that the differential signal 102 is very weak. The signal amplifying module 130 is configured to amplify the differential signal 102 to improve the accuracy of the wave signal 101.

Accordingly, all differential signals 102 passing through the signal amplifying module 130 will be amplified by the signal amplifying module 130, wherein the differential signal 102 contains ultra-high frequency signals, high frequency signals, and low frequency signals, and very low frequency signals. The amplified differential signal 102 is arranged to pass through the signal converting module 120 to form the wave signal 101, wherein the signal converting module 120 is configured to select the wave signal 101 in the specific frequency range segment to obtain the motion characteristics of the object in detection area.

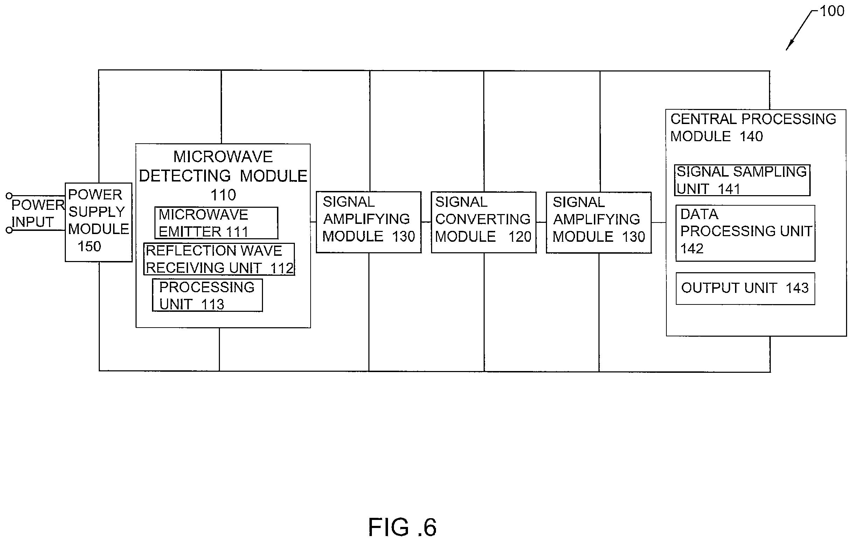

As shown in Fig. 6, the microwave detection device 100 of a second embodiment illustrates an alternative mode of the first embodiment of the present invention, wherein there are two signal amplifying modules 130. One of the signal amplifying modules 130, as the first signal amplifying module, is arranged to amplify the differential signal 102, wherein after the differential signal 102 is amplified, the amplified differential signal 102 is converted into the wave signal 101 via the signal converting module 120. Then, the wave signal 101 is amplified by another signal amplifying module 130, as the second signal amplifying module, to further enhance the accuracy of the microwave detection device 100.

According to the second embodiment, it should be understood by those skilled in the art that the signal amplifying module 130 and the signal converting module 120 may be integrated with each other to form a single module. In other words, the single module can be arranged to amplify and convert the differential signal 102 into the wave signal 101 at the same time so as to obtain the wave signal 101. For example, but not limited to, the module is implemented as an operational amplifier.

It is worth mentioning that the type of the signal amplifying module 130 is not limited, wherein the signal amplifying module 130 can be implemented as the amplifier constructed with at least a capacitor, at least a resistor, at least an inductor, and at least an operational amplifier. For example, but not limited to, the signal amplifying module 130 can be configured as a DC amplification module with one or more amplification power levels, an AC amplification module with one or more amplification power levels, or the combination thereof. The signal amplifying module 130 can perform single-stage or multi-stage amplification for the differential signal 102 and the wave signal 101.

As shown in FIG. 1, the microwave detection device 100 further comprises a central processing module 140 operatively connected to the signal converting module 120, wherein the central processing module 140 is arranged to acquire the wave signal 101 and to perform an analysis process on the wave signal 101 so as to determine the specific movement characteristic corresponding to the wave signal 101. Particularly, each parameter of the wave signal 101, such as but not limited to a wave frequency, a wave amplitude, and a duration, will represent the movement characteristics of the object within the detection area. The central processing module 140 is arranged to analyze the parameters of the wave signal 101 in order to determine specific movement characteristics of the object in the detection area, and/or distinguish different movement characteristics of the object in the detection area. In other words, it can more accurately acquire and/or distinguish the movement of the human body, limb movement, respiratory movement, and heartbeat movement, and at the same time, it can eliminate other interferences in the environment, such as filtering vibrations from air conditioners or exhaust fans, and malfunctions caused by animal, wind and/or rain.

Preferably, the central processing module 140 is arranged to determine different movement characteristics according to the wave frequency and/or the wave amplitude of the wave signal 101 to enhance the accuracy of the microwave detection device 100. In one example, the microwave detection device 100 is used for detecting movement characteristics of the user. The walking speed of the human being is about 0.3-1 meter per second, the rhythm is about 0.5-1 times per second, the fine body movement, such as head down, head up, body lean forward, body lean backward, body turning to the left, and body turning to the right, happens at every 0.5 second or more. Usually, during normal breathing, the human body may have some body micro-movements. When the signal converting module 120 selects and sets the wave signal 101 within the specific frequency range within 1 Hz, the selected wave signal 101 may correspond to the respiratory movement and the limb movement of the human body. However, there is a great difference between the amplitude of the chest expansion and the abdominal undulation movement and the amplitude of the limb movements, such as head down movement, head up movement, lean forward movement, lean backward movement, body turning to the left, body turning to the right, and etc.. Therefore, different movement characteristics can be determined based on the wave frequency and/or the wave amplitude of the wave signal 101. Alternatively, the central processing module 140 determines different movement characteristics according to different phase differences of the wave signal 101.

Preferably, in another embodiment, the central processing module 140 is arranged to determine different movement characteristics according to the time and/or the change period of the wave signal 101. The cycle of the wave signal 101 is regular for the continuous chest expansion due to breathing and abdominal undulations. The cycle of the wave signal 101 is irregular for head down movement, head up movement, lean forward movement, lean backward movement, body turning to the left, body turning to the right, and etc.. Therefore, different movement characteristics can be determined according to the duration and change period of the wave signal 101.

In another embodiment, the central processing module 140 is arranged to determine different movement characteristics based on the amplitude variation of the wave signal in one cycle. For example, when the specific frequency range of the signal converting module 120 of the microwave detection device 100 is set less than or equal to 25 Hz, the wave signal 101 can correspond to body movement, fine movement, respiratory movement, and heartbeat movement of the human body. Within the same period, the amplitude changes of the body movement, fine movement, respiratory movement, and heartbeat movement of the human body are different. Therefore, the amplitude of the wave signal 101 can be analyzed to distinguish different movement characteristics.

Alternatively, when the microwave detection device 100 is used for detecting a high-speed moving object in the detection area, such as but not limited to, a high speed vehicle or raining. The parameters, such as wave frequency, wave amplitude, time duration, and the like of the wave signal 101 for the high speed vehicle or raining are totally different from the parameters, such as wave frequency, wave amplitude, time duration, and the like of the wave signal 101 for the body movement, fine movement, respiratory movement, and heartbeat movement of the human body. The central processing module 140 is arranged to analyze and compare one or more parameters of different wave frequency, wave amplitude, time duration, and the like of the wave signal 101, and is arranged to distinguish movement characteristics of different objects, so as to further obtain the movement characteristics of target objects in detection area.

As shown in Fig. 1, the central processing module 140 comprises a signal sampling unit 141, a data processing unit 142, and an output unit 143, wherein the signal sampling unit 141, the data processing unit 142, and the output unit 143 are operatively connected with each other. The signal sampling unit 141 is operatively connected to the signal converting module 120, wherein the signal sampling unit 141 is arranged to acquire the wave signal 101. The data processing unit 142 allows different algorithms and programs to be selected according to different target requirements to analyze specific movement characteristics corresponding to the wave signal 101, in order to output the analysis result from the output unit 143.

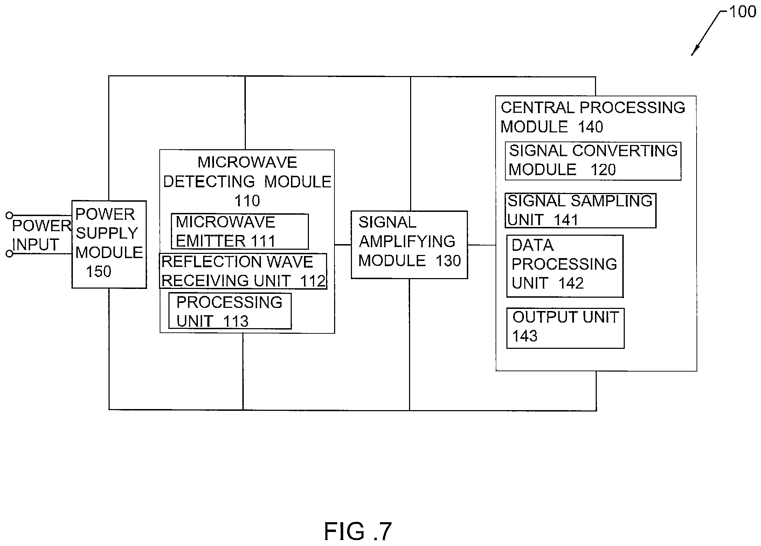

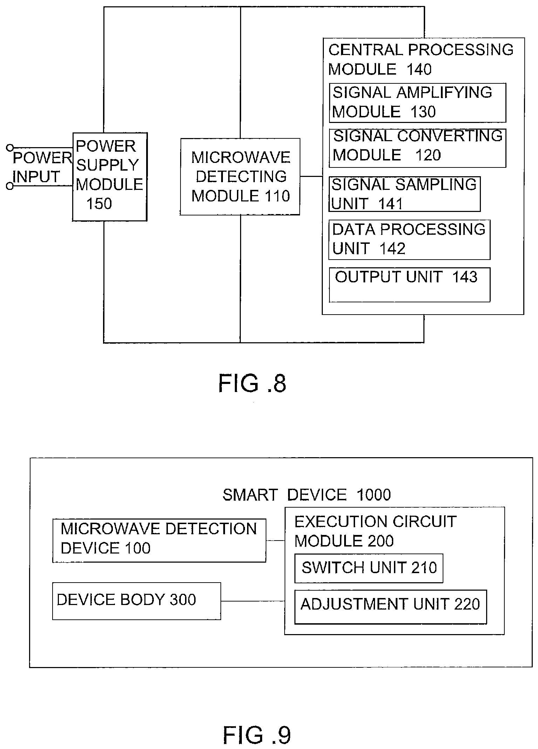

As shown in Fig. 1, in one embodiment, the signal sampling unit 141, the data processing unit 142, and the output unit 143 of the central processing module 140 of the microwave detector 100 are integrally formed with each other to form an integrated module. Alternatively, the signal amplifying module 130, and the signal sampling unit 141, the data processing unit 142, and the output unit 143 of the central processing module 140 are integrally formed with together to form an integrated module. Alternatively, as shown in Fig. 7, the signal converting module 120, and the signal sampling unit 141, the data processing unit 142, and the output unit 143 of the central processing module 140 are integrally formed with together to form an integrated module. Alternatively, as shown in Fig. 8, the signal converting module 120, the signal amplifying module 130, and the signal sampling unit 141, the data processing unit 142, and the output unit 143 of the central processing module 140 are integrally formed with together to form an integrated module. Alternatively, the microwave detecting module 110 and the signal amplifying module 130 are integrally formed with together to form an integrated module. Alternatively, the microwave detecting module 110, the signal amplifying module 130, and the signal converting module 120 are integrally formed with together to form an integrated module. Alternatively, the microwave detecting module 110, the signal converting module 120, the signal amplifying module 130, and the signal sampling unit 141, the data processing unit 142, and the output unit 143 of the central processing module 140 are integrally formed with together to form an integrated module.

The microwave detection device 100 further comprises a power supply module 150 electrically connected to the microwave detecting module 110, the signal converting module 120, the signal amplifying module 130, and the central processing module 140. Accordingly, the power supply module 150 is arranged to supply electric power to the microwave detecting module 110, the signal converting module 120, the signal amplifying module 130, and the central processing module 140.

The microwave detection device 100 is able to incorporate with a smart device 1000, such that the smart device 1000 is enabled to be automatically adjusted based on the movement characteristics of the objects within the detection area to provide intelligent services. Particularly, as shown in Fig. 9, the smart device 1000 is embodied to comprise at least one microwave detection device 100, an execution circuit module 200, and a device body 300, wherein the execution circuit module 200 is operatively connected to the microwave detection device 100. The execution circuit unit 200 controls the operation of the device body 300 in response to the analysis result output by the central processing unit 140 of the microwave detection device 100. The smart device 1000 is operated to provide personalized and intelligent services according to the need of the user under the desired environment.

Preferably, the execution circuit module 200 comprises a switch unit 210 and an adjustment unit 220, wherein the switch unit 210 and the adjustment unit 220 are operatively connected to the device body 300. The switch unit 210 is arranged to control the device body 300 between in a switch-on mode or a switch-off mode according to the analysis result output by the output unit 143 of the central processing module 140. The adjusting unit 200 is configured according to the analysis result output via the output unit 143 to adjust the operating parameters of the device body 300 in the switch-on mode. For example, the device body 300 is embodied as a light fixture, wherein the execution circuit module 200 is arranged to switch on and off the light fixture, to adjust the brightness of the light, to adjust the color tone of the light, and/or to adjust the direction of the light source according to the movement characteristics of the human body from the analysis result.

It is worth mentioning that the application of the device body 300 should not be limited. For example, but not limited to, the device body 300 can be implemented as an electronic device such as an air conditioner, an audio system, an automatic curtain, or the like. The execution circuit module 200 is able to control the on-and-off operations of the air conditioner, the audio system, and the automatic curtain according to the movement characteristics of the human body in the detection area. For example, the execution circuit module 200 is able to adjust the temperature setting of the air conditioner, the direction of the air outlet, the fan speed, and etc., or to adjust the music volume, the type of music played, and etc., or to control the open-close movement of the curtain to select the shading area thereof, and etc.

Accordingly, the present invention further provides a detection method by operating the microwave detection device 100 as embodied above, wherein the detection method comprises the following steps.
(a) Convert the differential signal 102 to the wave signal 101, wherein the wave signal 101 corresponds to a movement characteristic of an object within the detection area.
(b) Process the wave signal 101 to acquire the movement characteristic of the object in the detection area.

Accordingly, before the step (a), the detection method further comprises the steps of:
transmitting or emitting the detection wave to the detection area and receiving the reflected wave corresponding to the detection wave; and
outputting the differential signal 102 according to a frequency and/or phase difference of the detection wave and the reflected wave.

According to the preferred embodiment, in the step (a), the differential signal 102 is converted to the wave signal 101 based on at least a property change of the differential signal 102. Preferably, the differential signal 102 is converted to the wave signal 101 based on the amplitude change of the differential signal 102. Preferably, the differential signal 102 is converted to the wave signal 101 based on the phase change of the differential signal 102. Preferably, the differential signal 102 is converted to the wave signal 101 based on the change in pulse width of the differential signal 102. Preferably, the differential signal 102 is converted to the wave signal 101 based on the frequency change of the differential signal 102.

According to the preferred embodiment, in step (a), the differential signal 102 is converted to the wave signal 101 by using an envelope filtering method based on pulse amplitude. Alternatively, in step (a), the differential signal 102 is converted to the wave signal 101 by means of a pulse width based integration method.

According to the detection method, in the step (a), the movement characteristic of the object in the detection area is acquired by selecting the wave signal 101 in a specific frequency range. Preferably, the wave signal 101 is selected within a specific frequency range of 25 Hz, wherein the selected wave signal 101 is in an "electromagnetic wave free" segment, such that the signal amplifying module 130 is able to amplify the wave signal 101 in a relatively large magnification level to obtain the accurate and useful wave signal 101. Preferably, the specific frequency range of the wave signal 101 is selected and limited to be 50 Hz or less, in order to filter the wave signal 101 at other frequency segments. Preferably, for detecting the respiratory movement of the human body, the specific frequency range of the wave signal 101 is selected and limited to be 1 Hz or less in order to filter the wave signal 101 at other frequency segments. Preferably, for detecting the heartbeat movement of the human body, the specific frequency range of the wave signal 101 is selected and limited to be 1 Hz - 3 Hz in order to filter the wave signal 101 at other frequency segments.

Preferably, according to the detection method, the analog filter is used to convert the differential signal 102 into the wave signal 101 and, at the same time, to select the wave signal 101 in the specific frequency range. Preferably, according to the detection method, the peak value and the average value of the differential signal 102 are extracted by the digital filter by using a filtering algorithm, wherein the peak value or the average value is then combined together to form the wave signal 101.

According to a preferred embodiment, before the step (a), the detection method further comprises a step of amplifying the differential signal 102 to amplify weak differential signal 102, such that the weak difference signal 102 can also be accurately identified and calculated, so as to enhance the accuracy of the wave signal 101.

According to a preferred embodiment, after the step (a), the detection method further comprises a step of amplifying the fluctuation signal 101 to accurately acquire the specific movement of the object in the detection area. In addition, the selected wave signal 101 is in the "electromagnetic wave free" segment, such that the signal amplifying module 130 is able to amplify the wave signal 101 in a relatively magnification level to obtain an accurate and useful wave signal 101.

Preferably, in the step (b), different movement characteristics are determined according to the wave frequency and/or the wave amplitude of the wave signal 101, to enhance the accuracy of the microwave detection device 100. Preferably, in the step (b), different movement characteristics are determined according to different phase differences of the wave signal 101.

Preferably, in the step (b), different movement characteristics are determined according to the time duration of the wave signal 101.

Preferably, in the step (b), different movement characteristics are determined according to the amplitude change of the wave signal 101 in one cycle to accurately acquire specific movement characteristics of the object in the detection area.

One skilled in the art will understand that the embodiment of the present invention as shown in the drawings and described above is exemplary only and not intended to be limiting.

It will thus be seen that the objects of the present invention have been fully and effectively accomplished. The embodiments have been shown and described for the purposes of illustrating the functional and structural principles of the present invention and is subject to change without departure from such principles. Therefore, this invention includes all modifications encompassed within the spirit and scope of the following claims.

## Claims

1. A microwave detection device for detecting one or more movement characteristics of at least one object in a detection area thereof, comprising:
a microwave detecting module generating a differential signal; and
a signal converting module, operatively connected to said microwave detecting module, converting said difference signal into a wave signal, wherein a specific frequency range of said wave signal is selected and limited to be analyzed for determining the one or more movement characteristics of the at least one object in the detection area.

2. The microwave detection device, as recited in claim 1, wherein said microwave detecting module is arranged to emit a detection wave in the detecting area, wherein a reflected wave is formed when the detection wave is reflected by the at least one object in the detecting area, wherein said differential signal is outputted by said microwave detecting module in response to at least one of a frequency difference between said detection wave and said reflected wave and a phase difference between said detection wave and said reflected wave.

3. The microwave detection device, as recited in claim 1, wherein said signal converting module is arranged to convert said differential signal into said wave signal in response to at least a property change thereof for determining the one or more movement characteristics of the at least one object in the detection area, wherein said property change of said wave signal is at least one of an amplitude change of said wave signal, a phase change of said wave signal, a change in a pulse width of said wave signal, and a frequency change of said wave signal.

4. The microwave detection device, as recited in claim 1, wherein said signal converting module is at least one of an analog filter and a digital filter.

5. The microwave detection device, as recited in claim 1, further comprising at least one signal amplifying module operatively connected to at least one of said microwave detecting module and said signal converting module, wherein said differential signal is amplified by said signal amplifying module and is then converted into said wave signal by said signal converting module, wherein at least one of said signal amplifying module amplifies said differential signal and at least one of said signal amplifying module amplifies said wave signal.

6. The microwave detection device, as recited in claim 1, further comprising a central processing module operatively connected to said signal converting module, wherein said central processing module processes and analyzes said wave signal for determining the one or more movement characteristics of the at least one object in the detection area through said wave signal in response to at least one of a wave frequency of said wave signal, a wave amplitude of said wave signal, different phase differences of said wave signal, a time duration of said wave signal, and an amplitude change of said wave signal in one cycle, wherein said central processing module comprises a signal sampling unit, a data processing unit and an output unit operatively connected with each other, wherein said signal sampling unit, said data processing unit and said output unit are integrated to form a single unit.

7. The microwave detection device, as recited in claim 1, wherein said specific frequency range is set as 25 Hz or less to set said wave signal in an "electromagnetic wave free" segment.

8. The microwave detection device, as recited in claim 1, wherein said specific frequency range is set as 1 Hz or less for detecting a respiratory movement of a human body.

9. The microwave detection device, as recited in claim 1, wherein said specific frequency range is set between 1-3 Hz for detecting a heartbeat movement of a human body.

10. A detection method for detecting one or more movement characteristics of at least one object within a detection area of a microwave detection device, comprising the steps of:
(a) converting a differential signal to a wave signal via a signal converting module; and
(b) processing said wave signal to acquire the one or more movement characteristic of the at least one object in the detection area, wherein a specific frequency range of said wave signal is selected and limited to be analyzed for determining the one or more movement characteristics of the at least one object in the detection area.

11. The detection method, as recited in claim 10, before the step (a), further comprising the steps of:
via a microwave detecting module, emitting a detection wave to said detection area and receiving a reflected wave corresponding to said detection wave; and
outputting said differential signal via said microwave detecting module in response to at least one of a frequency difference between said detection wave and said reflected wave and a phase difference between said detection wave and said reflected wave.

12. The detection method, as recited in claim 10, wherein the step (a) further comprises a step of converting said differential signal into said wave signal in response to a property change of said signal converting module for determining the one or more movement characteristics of the at least one object in the detection area, wherein said property change of said wave signal is at least one of an amplitude change of said wave signal, a phase change of said wave signal, a change in a pulse width of said wave signal, and a frequency change of said wave signal.

13. The detection method, as recited in claim 10, wherein the step (a) further comprises a step of converting said differential signal to said wave signal selectively by using an envelope filtering method based on pulse amplitude or by means of a pulse width based integration method.

14. The detection method, as recited in claim 10, before the step (a), further comprising a step of amplifying said differential signal, and, after the step (a), further comprising a step of amplifying said wave signal.

15. The detection method, as recited in claim 10, wherein the step (b) further comprises a step of determining the different movement characteristics of the at least one object in the detection area in response to one of a wave frequency, of said wave signal, a wave amplitude of said wave signal, a time duration of said wave signal, and an amplitude change of said wave signal in one cycle.

16. The detection method, as recited in claim 10, wherein said specific frequency range is set as 25 Hz or less to set said wave signal in an "electromagnetic wave free" segment.

17. The detection method, as recited in claim 10, wherein said specific frequency range is set as 1 Hz or less for detecting a respiratory movement of a human body.

18. The detection method, as recited in claim 10, wherein said specific frequency range is set between 1-3 Hz for detecting a heartbeat movement of a human body.
